(19) Europäisches Patentamt
European Patent Office
Office européen des brevets

(11) Publication number : **0 581 442 A2**

(12) **EUROPEAN PATENT APPLICATION**

(21) Application number : **93305012.2**

(51) Int. Cl.[5] : **A61K 7/08,** A61K 7/06

(22) Date of filing : **28.06.93**

(30) Priority : **30.06.92 US 906978**

(43) Date of publication of application :
**02.02.94 Bulletin 94/05**

(84) Designated Contracting States :
**DE FR GB IT**

(71) Applicant : **GENERAL ELECTRIC COMPANY**
**1 River Road**
**Schenectady, NY 12345 (US)**

(72) Inventor : **Thimineur, Raymond Joseph**
**162 Willow Lane**
**Scotia, New York 12302 (US)**
Inventor : **Stoklosa, Stanley John**
**319 Front Street, Apartment 2B**
**Schenectady, New York 12305 (US)**

(74) Representative : **Pratt, Richard Wilson**
**London Patent Operation G.E. Technical Services Co. Inc. Essex House 12/13 Essex Street**
**London WC2R 3AA (GB)**

(54) **Shampoo composition.**

(57) A novel shampoo composition having improved silicone deposition and conditioning properties is disclosed which comprises a quaternary imidazolinium compound, and preferably an alkylene glycol mono or di stearate.

**EP 0 581 442 A2**

The present invention relates to a novel shampoo composition. More particularly the present invention relates to a novel shampoo composition having enhanced silicone deposition for improved conditioning properties. Most particularly the present invention relates to a shampoo composition comprising a quaternary imidazolinium compound, optionally in combination with an alkylene glycol mono- or di-stearate compound.

## BACKGROUND OF THE PRESENT INVENTION

A variety of 2 in 1 shampoo compositions are disclosed in the prior art. These 2 in 1 shampoo compositions are desirable since they combine aspects of cleansing and conditioning hair. The preparation of a 2 in 1 conditioning shampoo has proved to be difficult due to the inherent incompatibility between the surfactants and the conditioning agents.

Exemplary of the conditioning shampoos disclosed in the prior art are those set forth in Oh et al., United States Patent No. 4,704,272; Bolich, Jr. et al., United States Patent No. 4,788,006; and Duvel, United States Patent No. 5,034,218. These patents disclose the use of various quaternary ammonium salts in the shampoo compositions. However, none of these compositions provide the conditioning properties that are desired by consumers. It would therefore represent a notable advance in the state of the art if a new composition could be developed having improved conditioning properties over the prior art compositions.

To this end, Applicants have now developed a shampoo composition comprising a quaternary imidazolinium compound, optionally in combination with an alkylene glycol mono- or di- stearate compound, which provides dramatically improved conditioning properties. Such improvements are clearly shown in the working examples of this specification.

## SUMMARY OF THE PRESENT INVENTION

According to the present invention there is provided a shampoo composition comprising (a) an anionic surfactant, a nonionic surfactant or a mixture thereof; (b) a suspending agent; (c) a nonvolatile silicone; (d) a quaternary imidazolinium compound; and (e) water.

In preferred embodiments, the shampoo compositions of the present invention further comprise an alkylene glycol mono- or di- stearate component in addition to the imidazolinium compound.

Also according to the present invention there is provided a method of shampooing hair comprising applying more than about 0.1 g of a shampoo composition of the present invention to hair that has been wet and then rinsing out.

## DETAILED DESCRIPTION OF THE PRESENT INVENTION

The first component of the shampoo compositions of the present invention are anionic or nonionic surfactants (a) . The surfactant is generally present in the compositions of the present invention in an amount ranging from about 5 to about 50%, more preferably from about 10 to about 30%, based on the total weight of the composition. These surfactants can include all of those known to persons of ordinary skill in the art.

Typical anionic surfactants for use in the present invention include alkyl and alkyl ether sulfates. These materials have the respective formulae $ROSO_3M$ and $RO(C_2H_4O)_xSO_3M$ wherein R is alkyl or alkenyl of from about 10 to about 20 carbon atoms, x is 1 to 10, and M is a water-soluble cation such as ammonium, sodium, potassium or triethanolamine. The alkyl ether sulfates useful in the present invention are condensation products of ethylene oxide and monohydric alcohols having from about 10 to about 20 carbon atoms. Preferably, R has from 14 to 18 carbon atoms in both the alkyl and alkyl ether sulfates. The alcohols can be derived from fats, e.g., coconut oil or tallow, or can be synthetic. Lauryl alcohol and straight chain alcohols derived from coconut oil are preferred herein. Such alcohols are reacted with 1 to 10, and especially 3, molar proportions of ethylene oxide and the resulting mixture of molecular species, having, for example, an average of 3 moles of ethylene oxide per mole of alcohol, is sulfated and neutralized.

Specific examples of alkyl ether sulfates of the present invention are sodium coconut alkyl trioxyethylene sulfate; lithium tallow alkyl trioxyethylene sulfate; and sodium tallow alkyl hexaoxyethylene sulfate. Highly preferred alkyl ether sulfates are those comprising a mixture of individual compounds, said mixture having an average alkyl chain length of from about 12 to about 16 carbon atoms and an average degree of ethoxylation of from about 1 to about 4 moles of ethylene oxide. Such a mixture also comprises from about 0 to about 20% by weight $C_{12-13}$ compounds; from about 60 to 100% by weight of $C_{14-15-16}$ compounds; from about 0 to about 20% by weight of $C_{17-18-19}$ compounds, from about 3 to about 30% by weight of compounds having a degree of ethoxylation of 0, from about 45 to about 90% by weight of compounds having a degree of ethoxylation of

from 1 to 4; from about 10 to about 25% by weight of compounds having a degree of ethoxylation of from 4 to 9; and from about 0.1 to about 16% by weight of compounds having a degree of ethoxylation of greater than 8.

Additional examples of anionic synthetic detergents which come within the terms of the present invention are the reaction products of fatty acids esterified with isothionic acid and neutralized with sodium hydroxide wherein, for example, the fatty acids are derived from coconut oil; sodium or potassium salts of fatty acid amides of methyl lauride in which the fatty acids, for example, are derived from coconut oil. Other anionic synthetic detergents of this variety are set forth in United States Patent Nos. 2,486,921; 2,486,922 and 2,396,278.

Still other synthetic detergents include the class designated as succinamates. This class includes such surface active agents as disodium N-octadecylsulfosuccinamate; tetrasodium N-(1,2-dicarboxyethyl)-N-octadecylsulfosuccinamate; diamyl ester of sodium sulfosuccinic acid; dihexyl ester of sodium sulfosuccinic acid; dioctyl ester of sodium sulfosuccinic acid.

Other suitable anionic detergents utilizable herein are olefin sulfonates having from about 12 to about 24 carbon atoms. The term "olefin sulfonates" is used herein to mean compounds which can be produced by the sulfonation of α-olefin by means of uncomplexed sulfur trioxide, followed by neutralization of the acid reaction mixture in condition such that any sulfones which have been formed in the reaction are hydrolyzed to give the corresponding hydroxy-alkanesulfonates. The sulfur trioxide can be liquid or gaseous, and is usually, but not necessarily, diluted by inert diluents, for example by liquid $SO_2$, chlorinated hydrocarbons, etc., when used in the liquid form, or by air, nitrogen, gaseous $SO_2$, etc., when used in the gaseous form.

The α-olefins from which the olefin sulfonates are derived are mono-olefins having from 12 to 24 carbon atoms, preferably from 14 to 16 carbon atoms. Preferably, they are straight chain olefins. Examples of suitable 1-olefins include 1-dodecene; 1-tetradecene; 1-hexadecene; 1-octadecene; 1-eicosene and 1-tetracosene.

In addition to the true alkene sulfonates and a proportion of hydroxy-alkanesulfonates, the olefin sulfonates can contain minor amounts of other materials, such as alkene disulfonates, depending upon the reaction conditions, proportion of reactants, the nature of the starting olefins and impurities in the olefin stock and side reactions during the sulfonation process.

A specific α-olefin sulfonate mixture of the above-type is described more fully in the United States Patent No. 3,332,880.

Another class of anionic organic detergents are the β-alkyloxy alkane sulfonates. These compounds have the following formula:

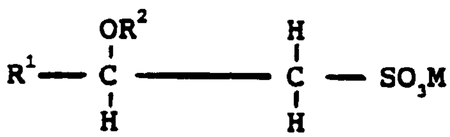

where $R^1$ is a straight chain alkyl group having from 6 to 20 carbon atoms, $R^2$ is a lower alkyl group having from 1 to 3 carbon atoms, and M is water-soluble cation as hereinbefore described.

Specific examples of β-alkyloxy-alkane-1-sulfonates, or alternatively 2-alkyloxy-alkane-1-sulfonates, having low hardness (calcium ion) sensitivity useful herein to provide superior cleaning levels under household washing conditions include: potassium-β-methoxydecanesulfonate, sodium 2-methoxytridecanesulfonate, potassium 2-ethoxytetradecylsulfonate, sodium 2- isopropoxyhexadecylsulfonate, lithium 2-t-butoxytetradecylsulfonate, sodiummethoxyoctadecylsulfonate, and ammonium-n-propoxydodecylsulfonate.

Another suitable class of anionic surfactants are the water-soluble salts of the organic, sulfuric acid reaction products of the general formula:

$$R^3 - SO_3 - M$$

wherein $R^3$ is chosen from the group consisting of a straight or branched chain, saturated aliphatic hydrocarbon radical having from 8 to 24, preferably from 12 to 18, carbon atoms; and M is a cation. Important examples are the salts of an organic sulfuric acid reaction product of a hydrocarbon of the methane series, including iso-, neo-, ineso-, and n-paraffins, having from 8 to 24 carbons atoms, preferably from 12 to 18 carbon atoms and a sulfonating agent, e.g., $SO_3$, $H_2SO_4$, oleum, obtained according to known sulfonation methods, including bleaching and hydrolysis. Preferred are alkali metal and ammonium sulfonated $C_{12-18}$-n-paraffins.

Many additional nonsoap synthetic anionic surfactants are described in McCutcheon's, Detergents and Emulsifiers, 1984 Annual, published by Allured Publishing Corporation. Also, United States Patent No. 3,929,678 discloses many other anionic as well as other surfactant types.

It is also contemplated herein that the above anionic surfactants can be employed with known non-ionic

surfactants; or that the non-ionic surfactants can be employed alone as component (a). Exemplary of useful non-ionic surfactants are alkanolamides and ethoxylated amides. These are well known to those of ordinary skill in the art and are available commercially.

Also contemplated for use in the present invention as component (a) are the amphoteric surfactants, including but not limited to amine oxides and betaine compounds. These are also available commercially.

The above-mentioned surfactants can be used alone or in combination in the shampoo compositions of the present invention. The alkyl sulfates, the ethoxylated alkyl sulfates and mixtures thereof are preferred for use herein.

The second component of the compositions of the present invention are the suspending agents (b). These materials are generally known to those of ordinary skill in the art. Two suitable materials are anionic polymers and xanthan gum, as well as other long chain materials.

The anionic polymers for use as suspending agents (b) in the present invention are the cross-linked anionic polymers of acrylic or methacrylic acid derivatives, for example, acrylic acid, the alkali metal and ammonium salts of acrylic acid, methacrylic acid, the alkali metal salts of methacrylic acid, acrylamide, methacrylamide, the N-alkyl substituted amides, the N-aminoalkylamides, and the corresponding N-alkylaminoalkyl substituted amides, the aminoalkyl acrylates, the aminoalkyl methacrylamides and the N-alkyl substituted aminoalkyl esters of either acrylic or methacrylic acids. These polymeric compositions may be the homopolymers or they may be copolymers with other copolymerizing monomers, such as ethylene, propylene, isobutylene, styrene, $\alpha$-methylstyrene, vinyl acetate, vinyl formate, alkyl ethers, acrylonitrile, methacrylonitrile, vinyl chloride, vinylidene chloride, the alkyl acrylates, the alkyl methacrylates, the alkyl maleates, and the alkyl fumarates, and other olefinic monomers copolymerizable therewith.

Another class of cross-linked anionic organic polymers are the polymers of vinyl sulfonic acid, and the copolymers of vinyl sulfonic acid with one or more polymerizable organic monomers, for example, vinyl chloride, acrylonitrile, styrene, vinyl acetate and other polymerizable mono-olefinic compounds. The sulfonic acid groups so introduced may be converted to sulfonic acid salts, acid amides or other electrolytic groupings. The copolymers of this type may involve the use of a plurality of sulfonic acid monomers and/or a plurality of the conventional comonomers as described.

The cross-linked anionic polymers should have a weight average molecular weight of at least about 50,000, preferably at least about 150,000 and more preferably at least about 1,000,000.

Suitable commercially available anionic polymers are the cross-linked acrylic acid polymers sold under the trademark Carbopol® by B.F. Goodrich Company. Particularly suitable are Carbopol® 1382 and Carbopol® 1342. These materials are further described in United States Patent Nos. 4,509,949; 3,915,921; and 2,798,053.

Xanthan gum is an agent which can be used in the present compositions to suspend the silicone fluid. This biosynthetic gum material is commercially available and is a heteropolysaccharide with a molecular weight of greater than 1 million. It contains D-glucose, D-mannose and D-gluconorate in the molar ratio of 2.8:2.0:2.0. The polysaccharide is partially acetylated with 4.7% acetyl. This information and other is found in Whistler, Roy L., ed., Industrial Gums-Polysaccharides and Their Derivatives, New York, Academic Press, 1973,. Kelco, a division of Merck & Co., Inc., offers xanthan gum as Keltrol®. The gum is present at a level of from about 0.2% to about 3%, preferably from about 0.6% to about 1.2% in the compositions of the present invention.

Other suitable suspending agents are alkanol amides of fatty acids, having from about 16 to about 18 carbon atoms. Preferred alkanol amides are stearic monoethanolamide, stearic diethanolamide, stearic monoisopropanolamide and stearic monoethanolamide stearate.

Still other suitable non-acyl derivative suspending agents are alkyl($C_{16-22}$)dimethyl amine oxides such as stearyl dimethyl amine oxide.

The suspending agent or mixture of agents is present at a level of from about 0.2% to about 5%, preferably from about 0.5% to about 2.5%. The suspending agent serves to assist in suspending the silicone material.

Particularly useful suspending agents (b) are xanthan gum, Carbopol® 1382 or hydroxycellulose materials.

Also present in the compositions of the present invention are nonvolatile silicones (c). The non-volatile silicone fluid may be either a polyalkyl siloxane, a polyaryl siloxane, a polyalkylaryl siloxane or a polyether siloxane copolymer and is present at a level ranging from about 0.1 to about 10.0%, preferably from about 0.5 to about 5%. Mixtures of these fluids may also be used and are preferred in certain embodiments. The dispersed silicone particles should also be insoluble in the shampoo matrix, i.e.

The nonvolatile polyalkyl siloxanes that may be used include for example, polydimethyl siloxanes with viscosities ranging from about 5 to about 1,500,000 centistokes at 25°C. These siloxanes are available commercially, such as from the General Electric Company as the Viscasil® series, and from Dow Corning Company as the Dow Corning 200 series. Preferably, the viscosity ranges from about 10,000 to about 600,000 centistokes.

The non-volatile polyalkylaryl siloxanes that may be employed in the compositions of the present invention are those such as polymethylphenylsiloxanes having viscosities of from about 15 to about 65 centistokes at 25°C. These siloxanes are available commercially, such as from Dow Corning as 556 Cosmetic Grade Fluid.

The polyether siloxane copolymers for use herein are those such as the Dow Corning DC-1248 fluid comprising a polypropylene oxide modified dimethylsiloxane. Further, ethylene oxide or mixtures of ethylene oxide and propylene oxide may be employed as the polyether component.

Mixtures of the above nonvolatile silicones with silicone gums may also be employed. These are known to those of ordinary skill in the art and are available commercially.

References disclosing suitable silicones are United States Patent Nos. 2,826,551; 4,364,837; and 3,964,500; and British Patent No. 849,433. Further silicones for use in the present invention are disclosed in Silicon Compounds distributed by Petrarch Systems, Inc. 1984.

Another component of the compositions of the present invention are quaternary imidazolinium compounds (d). These compounds are known to those of ordinary skill in the art and are available commercially as Varisoft® 475. A particularly preferred compound is methyl-1-tallow amido ethyl-2-tallow imidazolinium methylsulfate, commonly known by the CFTA name as Quaternium 27.

Water (e) is also included in the compositions of the present invention. Typically, from about 20 to about 95%, preferably from about 60 to about 85% of water is used in the shampoo compositions of the present invention.

Further, in other embodiments of the present invention, it has been found that the quaternary imidazolinium compound (d) can be used in conjunction with (f) a long chain acyl derivative, material or mixtures of such materials to obtain enhanced silicone conditioner deposition. Included are alkylene glycol esters of fatty acids having from about 16 to about 22 carbon atoms. Particularly useful are ethylene glycol monostearate, ethylene glycol distearate or a mixture thereof.

The shampoo compositions of the present invention further provide for the addition of other hair care additives (g). These components are employed to produce the commercially acceptable embodiments of the shampoo compositions.

Such conventional ingredients are well known to those skilled in the art, e.g. preservatives such as benzyl alcohol, methyl paraben, propyl paraben and imidazolidinyl urea; cationic surfactants such as lauryl trimethyl ammonium chloride, cetyl trimethyl ammonium chloride, stearyl-dimethyl benzyl ammonium chloride and di(partially hydrogenated tallow) dimethylammonium chloride; thickeners and viscosity modifiers such as a diethanolamine of a long chain fatty acid (e.g. Cocamide MEA), amine oxides, block polymers of ethylene oxide and propylene oxide such as Pluronic® F88 offered by BASF Wyandotte, fatty alcohols such as cetearyl alcohol, sodium chloride, sodium sulfate, polyvinyl alcohol and ethyl alcohol; pH adjusting agents such as mono sodium phosphate and disodium phosphate, citric acid, succinic acid, phosphoric acid, sodium hydroxide, sodium carbonate, etc.; perfumes; dyes; and sequestering agents such as disodium ethylenediamine tetraacetate. Such agents are generally used individually in amounts ranging from about 0.01% to about 10%, preferably from about 0.5 to about 5% by weight of the composition.

The pH of the shampoo compositions is not critical and may be in the range of from about 2.5 to about 10.

Particularly useful hair care additives (g) are fatty alcohols, panethol, botonicals, quaternized animal protein, which aid in producing a shampoo with conditioning properties.

The shampoo compositions of the are typically prepared by conventional means, i.e. by mixing the ingredients. A preferred method is a combination of high shear and low speed blending.

The shampoo compositions of the present invention can then be used in a method of shampooing hair comprising applying more than about 0.1 g of the shampoo compositions of the present invention to hair that has been wet and then rinsing the shampoo composition out of the hair.

## DESCRIPTION OF THE PREFERRED EMBODIMENTS

The following examples illustrate the present invention. They are not to be construed to limit the appended claims in any manner whatsoever.

The following procedure is employed for testing the various sample compositions for conditioning properties by determining the amount of silicone deposited on the hair.

5 g swatches of bleached white hair, 8 inch tresses from DeMeo Bros., Inc., New York, New York, are washed with a silicone free shampoo, Prell® Shampoo (Normal to Oily Hair) Procter & Gamble, Cincinnati, Ohio. The swatches are then rinsed thoroughly under warm tap water for 30 seconds to remove residual shampoo. The swatches are then hung to drip dry.

Each hair swatch is then treated with about 2 g of silicone conditioning shampoo by massaging the shampoo through the swatch for 30 seconds. The swatches are then gently rinsed under a 37°C tap water stream for 30 seconds. The treatment and rinse is then repeated. The treated swatches are then hung to air dry for from 12 to 24 hours.

A papain enzyme solution is prepared using papain powder, 98%, from Pfaltz and Bauer, Inc., according to the procedure of E.G. Gooch and G.S. Kohl, "Method to Determine Silicones on Human Hair by Atomic Absorption Spectroscopy," J. Soc. Cosmet. Chem., 39, 383-392 (1988).

0.30 ± 0.01 g of treated hair is then placed in a clean vial. To the vial is then added 10.0 g of the enzyme solution and the mixture is heated at 63 ± 3°C for 3 days. The mixture is then extracted and decanted twice with 7.0 and 3.0 ml of methyl iso-butyl ketone, MIBK Ultrapure Reagent, J.T. Baker, Inc. The extract is then analyzed by atomic absorption spectroscopy using appropriate standards and calibration (Perkin Elmer Z-5100 PC Atomic Absorption Spectrometer). Silicone deposition is reported either as ppm silicone in the MIBK solution or a ppm silicone on the hair.

In the following examples and tables, the parts by weight are reported based on percent active ingredients.

## EXAMPLE 1

A series of 2 in 1 shampoo formulations using Viscasil® 60000 as the silicone are prepared using several types of quaternary materials. All of the formulations passed 6 weeks at 45°C stability testing before being subjected to evaluation of hair deposition studies. Table 1 below sets forth the results, along with the compositional data.

Table 1

| Example | 1 | 1A* | 1B* | 1C* | 1D* |
|---|---|---|---|---|---|
| **Composition, pbw** | | | | | |
| Sodium Laureth Sulfate[a] | 10.0 | 10.0 | 10.0 | 10.0 | 10.0 |
| Lauramide DEA[b] | 4.0 | 4.0 | 4.0 | 4.0 | 4.0 |
| Carbopol® 1382[c] | 0.8 | 0.8 | 0.8 | 0.8 | 0.8 |
| Viscasil® 60000[d] | 2.0 | 2.0 | 2.0 | 2.0 | 2.0 |
| Varisoft® 475[e] | 1.0 | -- | -- | -- | -- |
| Arquad® 316[f] | -- | -- | 1.0 | -- | -- |
| Adogen® 432CG[g] | -- | -- | -- | 1.0 | -- |
| Arquad® 18/50[h] | -- | -- | -- | -- | 1.0 |
| 25% Sodium Hydroxide[i] | X | X | X | X | X |
| 33% Citric Acid[j] | Y | Y | Y | Y | Y |
| Water | Z | Z | Z | Z | Z |
| **Properties** | | | | | |
| Silicone, ppm MIBK | 10.2 | 4.3 | 7.9 | 6.7 | 3.4 |
| Silicone, ppm hair | 340 | 143 | 263 | 223 | 113 |

* = Comparative Example
a = Primary Surfactant, available from Stepan Company
b = Secondary Surfactant, available from Stepan Company
c = Suspending Agent for Silicone, available from BF Goodrich Company
d = Conditioning Agent, available from General Electric Company
e = Quaternary Imidazolinium compound, available from Sherex Chemical Company, Inc.
f = Tri alkyl quaternary ammonium compound, available from Akzo Chemicals, Inc.
g = Di alkyl quaternary ammonium compound, available from Sherex Chemical Company, Inc.
h = Mono alkyl quaternary ammonium compound, available from Akzo Chemicals, Inc.
i = Neutralizing agent
j = Final pH adjuster
X = Sufficient amount to activate Carbopol at pH=7.5
Y = Sufficient amount to adjust final formulation to pH=6.0
Z = Sufficient amount to total 100 parts by weight

EXAMPLES 2-4

A second series of formulations are prepared with the addition of ethylene glycol monostearate and ethylene glycol distearate.

The results along with compositional data are set forth in Table 2 below.

TABLE 2

| Example | 2 | 3 | 4 |
|---|---|---|---|
| **Composition, pbw** | | | |
| Sodium Laureth Sulfate[a] | 10.0 | 10.0 | 10.0 |
| Lauramide DEA[b] | 4.0 | 4.0 | 4.0 |
| Varisoft® 475[c] | 1.0 | 1.0 | -- |
| Viscasil® 60000[d] | 2.0 | 2.0 | 2.0 |
| Carbopol® 1382[e] | 0.8 | 0.8 | 0.8 |
| EGMS[f] | 1.0 | -- | 1.0 |
| EGDS[g] | -- | 1.0 | -- |
| 25% Sodium Hydroxide[h] | X | X | X |
| 33% Citric Acid | Y | Y | Y |
| Water | Z | Z | Z |
| **Properties** | | | |
| Silicone, ppm MIBK | 17.5 | 15.7 | 6.0 |
| Silicone, ppm hair | 583 | 523 | 200 |

[a] = Primary Surfactant, available from Stepan Company
[b] = Secondary Surfactant, available from Stepan Company
[c] = Quaternary Imidazolinium compound, available from Sherex Chemical Co., Inc.
[d] = Conditioning Agent, available from General Electric Company
[e] = Suspending Agent for Silicone, available from BF Goodrich Company
[f] = Ethylene glycol mono stearate
[g] = Ethylene glycol di stearate
[h] = Neutralizing agent
[i] = Final pH adjuster
X = Sufficient amount to activate Carbopol at pH=7.5
Y = Sufficient amount to adjust final formulation to pH=6.0
Z = Sufficient amount to total 100 parts by weight

EXAMPLES 5-7

A series of samples were tested which employed an ammonium derived surfactant as the primary surfactant instead of a sodium derived surfactant.

The results are set forth, along with compositional data, in Table 3 above.

**TABLE 3**

| Example | 5 | 6 | 7 |
|---|---|---|---|
| **Composition, pbw** | | | |
| Ammonium Laureth Sulfate[a] | 10.0 | 10.0 | 10.0 |
| Lauramide DEA[b] | 4.0 | 4.0 | 4.0 |
| Varisoft® 475[c] | 1.0 | 1.0 | 1.0 |
| Viscasil® 60000[d] | 2.0 | 2.0 | 2.0 |
| Carbopol® 1382[e] | 0.8 | 0.8 | -- |
| EGDS[f] | -- | 1.0 | 1.0 |
| 25% Sodium Hydroxide[g] | X | X | -- |
| 33% Citric Acid[h] | Y | Y | Y |
| Water | Z | Z | Z |
| Keltrol® T[i] | -- | -- | 0.75 |
| **Properties** | | | |
| Silicone, ppm MIBK | 4.6 | 9.4 | 9.1 |
| Silicone, ppm hair | 153 | 313 | 303 |

[a] = Primary Surfactant, available from Stepan Company
[b] = Secondary Surfactant, available from Stepan Company
[c] = Quaternary Imidazolinium compound, available from Sherex Chemical Co., Inc.
[d] = Conditioning Agent, available from General Electric Company
[e] = Suspending Agent for Silicone, available from BF Goodrich Company
[f] = Ethylene glycol di stearate
[g] = Neutralizing agent
[h] = Final pH adjuster
[i] = Grade of xanthan gum, available from Kelco division of Merck & Co., Inc.
X = Sufficient amount to activate Carbopol at pH=7.5
Y = Sufficient amount to adjust final formulation to pH=6.0
Z = Sufficient amount to total 100 parts by weight

## COMPARATIVE EXAMPLES E AND F

Two commercial products are purchased off the shelf and tested for silicone deposition on the hair using the same procedure as outlined above.

Commercial Sample E, a formulation according to United States Patent No. 4,704,272, contains nominal 3% high viscosity silicone, tricetyl ammonium chloride, xanthan gum and the usual surfactants and other embodiments found in commercial shampoos.

Commercial Sample F, a formulation according to United States Patent No. 5,034,218, contains nominal 2% high viscosity silicone, distearyl dimonium chloride and the usual surfactants and other embodiments found in commercial shampoos.

The following silicone deposition properties were found.

| | Silicone, ppm MIBK | Silicone, ppm hair |
|---|---|---|
| Example E | 1.7 | 57 |
| Example F | 1.2 | 40 |

It can be seen that the formulations of the present invention provide significantly improved silicone deposition over the commercial formulations of the prior art.

The above-mentioned patents and publications are hereby incorporated by reference.

Many variations of the present invention will suggest themselves to those skilled in the art in light of the above-detailed description. For example, other silicones such as Viscasil® 100000 or Viscasil® 300000, or blends of silicone fluids with gums can be employed in the shampoo compositions of the present invention. All such obvious modifications are within the full intended scope of the appended claims.

## Claims

1. A shampoo composition comprising :
   (a) an anionic surfactant, a nonionic surfactant or a mixture thereof;
   (b) a suspending agent;
   (c) a nonvolatile silicone;
   (d) a quaternary imidazolinium compound; and
   (e) water.

2. A shampoo composition as defined in Claim 1 wherein said surfactant (a) comprises an anionic surfactant.

3. A shampoo composition as defined in Claim 2 wherein said anionic surfactant (a) is selected from the group consisting of alkyl sulfates, ethoxylated alkyl sulfates, olefin sulfates, succinamates and beta-alkyloxyalkane sulfonates.

4. A shampoo composition as defined in Claim 1 wherein said suspending agent (b) comprises a cross-linked acrylic resin.

5. A shampoo composition as defined in Claim 1 wherein said suspending agent (b) comprises xanthan gum.

6. A shampoo composition as defined in Claim 1 wherein said quaternary imidazolinium compound (d) comprises methyl-1-tallow amido ethyl-2-tallow imidazolinium methyl sulfate.

7. A shampoo composition as defined in any preceding claim comprising from about 5 to about 50% of component (a), from about 0.1 to about 10% of component (b), from about 0.1 to about 4% of component (c), from about 0.2 to about 5% of component (d) and the remainder water (e).

8. A shampoo composition as defined in any preceding claim further comprising (f) an alkylene glycol mono or distearate.

9. A shampoo composition as defined in any preceding claim further comprising (g) a hair care additive.

10. A method of shampooing hair comprising applying more than about 0.1g of a shampoo composition according to any preceding claim to hair that has been wet and then rinsing out.